# EUROPÄISCHE PATENTSCHRIFT

(11) **EP 2 841 122 B1**
(45) Veröffentlichungstag und Bekanntmachung des Hinweises auf die Patenterteilung: **07.10.2015**
(21) Anmeldenummer: 13718340.6
(22) Anmeldetag: 25.04.2013
(51) Int. Cl.: A61M 1/10, A61B 5/0215

(54) **INTRAVASALE ROTATIONSBLUTPUMPE**
INTRAVASCULAR ROTARY BLOOD PUMP
POMPE À SANG ROTATIVE INTRA-VASCULAIRE

(30) Priorität: 27.04.2012 DE 102012207049
(43) Veröffentlichungstag der Anmeldung: 04.03.2015
(73) Patentinhaber: Abiomed Europe GmbH, 52074 Aachen (DE)
(72) Erfinder: SPANIER, Gerd, 52074 Aachen (DE); SIESS, Thorsten, 52074 Aachen (DE); KIRCHHOFF, Frank, 52074 Aachen (DE)
(74) Vertreter: Klunker . Schmitt-Nilson . Hirsch
(86) Internationale Anmeldenummer: PCT/EP2013/058642
(87) Internationale Veröffentlichungsnummer: WO 2013/160407

(56) Entgegenhaltungen:
- WO-A1-01/74419
- WO-A1-2011/039091
- US-A- 5 911 685
- US-A1- 2010 241 008
- US-B1- 6 176 822

## Beschreibung

Die Erfindung betrifft eine intravasale Rotationsblutpumpe mit ein oder mehreren Drucksensoren zum Messen von Drücken innerhalb des vaskulären Systems des Patienten, die für den Betrieb der Blutpumpe und/ oder für die Beurteilung des Gesundheitszustands des Patienten von Bedeutung sind.

Intravasale Rotationsblutpumpen dienen der temporären Herzunterstützung und stellen eine interessante Alternative zu den herkömmlichen intraaortalen Ballonpumpen (IABP) dar. Derartige Blutpumpen werden perkutan beispielsweise in die Femoralarterie eingeführt und durch das Gefäßsystem des Körpers hindurch geführt, um beispielsweise die Pumparbeit im Herzen zu unterstützen oder zu ersetzen. Aus der US 5,911,685 ist eine intravasale Rotationsblutpumpe bekannt, die eine Pumpeinrichtung und einen am proximalen Ende der Pumpeinrichtung angeschlossenen Katheter besitzt, durch den hindurch verschiedene Leitungen, beispielsweise Stromversorgungsleitungen für die Pumpeinrichtung, verlaufen. Die Pumpeinrichtung selbst umfasst einen Motorteil und einen am distalen Ende des Motorteils befestigten Pumpenteil. Das Pumpenteil umfasst ein rohrförmiges Pumpengehäuse, in dem ein Flügelrad dreht, welches auf einer aus dem Motorteil herausragenden Motorwelle sitzt. An das distale Ende des Pumpenteils schließt sich eine Strömungskanüle an, durch die hindurch im Betrieb der Blutpumpe Blut von der Pumpeinrichtung angesaugt oder bei umgekehrter Förderrichtung ausgestoßen wird. Während des Betriebs ragt die Pumpeinrichtung mit ihrer Strömungskanüle durch eine Herzklappenöffnung hindurch, um Blut mittels der Pumpeinrichtung durch die geöffnete Herzklappe hindurch fördern zu können. Desweiteren ist die Blutpumpe mit Drucksensoren außen am Pumpengehäuse und außen an der Strömungskanüle ausgestattet, um den Einlassdruck und den Auslassdruck zu ermitteln. Daten bezüglich des Einlass- und Auslassdrucks bilden zusammen mit dem Stromverbrauch des elektrischen Motors der Pumpeinrichtung eine Menge relevanter Informationen für die Funktion und Förderleistung der Pumpeinrichtung. Darüber hinaus kann man aus den gemessenen Drücken Rückschlüsse auf die Positionierung der Blutpumpe im Gefäßsystem ziehen. Außerdem lassen sich durch Vergleich des Differenzdrucks mit dem aktuellen Stromverbrauch des Motors lokale Zustände sowie Kavitation und Ansaugen feststellen.

In EP 1911484 A2 werden verschiedene Druckmesssysteme, die mit intraaortalen Ballonkathetern Verwendung finden, vorgestellt und deren Nachteile herausgestellt. Vorgeschlagen wird darin stattdessen die Verwendung von intraaortalen Ballonkathetern mit faseroptischen Drucksensoren. Der druckempfindliche Sensorkopf des faseroptischen Drucksensors endet in einer mit Flüssigkeit gefüllten Kammer, die gegenüber der Umgebung durch eine dünne Membran abgedichtet ist. Die dünne Membran bildet einen Teil des Gehäuses der Pumpeinrichtung und überträgt den Umgebungsdruck auf die Flüssigkeit innerhalb der Kammer. Der sich verändernde Druck innerhalb der Kammer wird mittels des Drucksensors detektiert. Durch die Lage des Sensorkopfs innerhalb der durch die flexible Membran abgeschlossenen, flüssigkeitsgefüllten Kammer wird der Drucksensor gegen Beschädigung beim Einführen und Platzieren der Herzunterstützungspumpe geschützt.

In WO 2011/039091 A1 wird die Verwendung von optischen Drucksensoren mit Lichtwellenleitern im Zusammenhang mit einer intravasalen Rotationsblutpumpe beschrieben. Auch hier befindet sich ein Sensorkopf außen am Gehäuse des Pumpenteils. Ein zweiter Drucksensor ist als separater Druckmesskatheter ausgebildet, der durch den eigentlichen Katheterschlauch hindurch verlegt ist, kurz vor der Pumpeinrichtung aus dem Katheterschlauch austritt und durch die Aortenklappe hindurch weit in den linken Ventrikel frei hineinragt. Der dort beschriebene optische Drucksensor arbeitet nach dem Fabri-Perot-Prinzip und wird auch im Zusammenhang mit der vorliegenden Erfindung bevorzugt verwendet. Der Sensorkopf eines solchen Drucksensors besitzt eine Kavität, die einerseits durch eine dünne, druckempfindliche Glasmembran abgeschlossen wird und in die andererseits das Ende einer Lichtleitfaser hineinragt. Die druckempfindliche Glasmembran verformt sich in Abhängigkeit von der Größe des auf den Sensorkopf einwirkenden Drucks. Durch die Reflexion an der Glasmembran wird das aus der Lichtleitfaser austretende Licht modulierend reflektiert und wieder in die Lichtleitfaser eingespeist. Am proximalen Ende der Lichtleitfaser befindet sich eine Auswerteeinheit mit integrierter CCD-Kamera, die das erhaltene Licht in Form eines Interferenzmusters auswertet. In Abhängigkeit hiervon wird ein druckabhängiges elektrisches Signal erzeugt.

Jedoch sind auch andere Drucksensoren im Zusammenhang mit der vorliegenden Erfindung geeignet, insbesondere andersartige faseroptische Drucksensoren. So beschreibt beispielsweise die US 6,398,738 B1 sowohl Drucksensoren vom Dehnungsmesstyp, insbesondere auf Basis von Halbleitermaterialien wie Silizium, als auch faseroptische Drucksensoren jeweils zur Verwendung mit intraaortalen Ballonkathetern. Erläutert werden unter anderem ein faseroptischer Drucksensor, bei dem Licht über eine erste Faser auf einen Spiegel geleitet und das von dem Spiegel reflektierte Licht über eine zweite Faser zurückgeführt und ausgewertet wird. Der Spiegel ist Teil eines Diaphragmas, das einseitig dem Blutdruck und auf der anderen Seite einem Referenzdruck ausgesetzt ist. Erläutert wird insbesondere aber auch eine Variante ohne das Erfordernis eines Referenzdrucks.

Die vorbeschriebenen Druckmesssysteme liefern - jedenfalls bei Anwendung in intravasalen Rotationsblutpumpen - nicht immer ausreichend aussagekräftige Messdaten. Insbesondere lassen sich hochfrequente physiologische Druckschwankungen nicht eindeutig von Störsignalen unterscheiden.

Aufgabe der vorliegenden Erfindung ist es daher, die Druckmessung für intravasale Rotationsblutpumpen zu verbessern.

Diese Aufgabe wird durch eine intravasale Rotationsblutpumpe mit den Merkmalen des Anspruchs 1 gelöst. In davon abhängigen Ansprüchen sind vorteilhafte Ausgestaltungen und Weiterbildungen der Erfindung angegeben.

Eine bevorzugte Ausführung einer erfindungsgemäßen intravasalen Rotationsblutpumpe sieht dazu vor, die distal am Katheter fixierte Pumpeinrichtung mit einem Drucksensor zu kombinieren, indem eine druckempfindliche Fläche des Drucksensors derart fest mit der Pumpeinrichtung verbunden wird, dass sie einerseits der Umgebung, deren Druck gemessen werden soll, ausgesetzt ist und andererseits orthogonal zu einer allgemeinen Längsachse der Rotationsblutpumpe ausgerichtet ist.

Es hat sich herausgestellt, dass sich mittels eines Drucksensors, dessen fest mit der Pumpeinrichtung verbundene druckempfindliche Fläche orthogonal zur Blutpumpenlängsachse ausgerichtet ist, auch hochfrequente physiologische Signale bis 250 Hz aus den Signaldaten ableiten lassen. Solche Informationen sind für die Diagnose über den Zustand oder Erholungszustand eines Herzens von erheblicher Bedeutung. Es wird vermutet, dass den Druckmessungen herkömmlicher Drucksensoren hochfrequente Störsignale überlagert sind, die von Unwuchten und anderen dynamischen Einflüssen beim Betrieb der Rotationsblutpumpe herrühren. Insbesondere haben Versuche gezeigt, dass sich intravasale Rotationsblutpumpen während des Betriebs in radialer Richtung, also quer zur allgemeinen Längsachse, hin- und herbewegen. Wird nun wie vorgeschlagen die druckempfindliche Fläche des Drucksensors orthogonal zur Längsachse ausgerichtet, so verursachen derartige Querbewegungen keinerlei Druckkräfte auf die druckempfindliche Fläche. Durch den Betrieb der Pumpeinrichtung verursachte Einflüsse auf das Druckmessergebnis, insbesondere im hochfrequenten Bereich, werden somit großteils eliminiert.

Die druckempfindliche Fläche kann beispielsweise die Glasmembran des Sensorkopfes des eingangs beschriebenen, nach dem Fabri-Perot-Prinzip arbeitenden Drucksensors sein. Sofern der Sensorkopf aber selbst nur den Druck in einer vorgelagerten Druckkammer misst, die von der Umgebung beispielsweise durch eine druckempfindliche Membran getrennt ist, so bildet diese vorgelagerte Membran die druckempfindliche Fläche des Drucksensors im Sinne der vorliegenden Erfindung. Denn es kommt auf diejenige druckübertragende Fläche an, die unmittelbar an die Umgebung angrenzt, deren Druck gemessen werden soll.

Als Drucksensor wird bevorzugt ein optischer Drucksensor mit einer Lichtleitfaser eingesetzt, bei der die druckempfindliche Fläche eine Membran ist und die Lichtleitfaser in einem Abstand zur Membran endet. Insoweit wird auf den Inhalt der WO 2011/039091 A1 Bezug genommen. Das bedeutet, dass der Sensorkopf des optischen Drucksensors nicht den Druck in einer vorgelagerten Druckkammer misst, sondern dem zu messenden Umgebungsdruck unmittelbar ausgesetzt ist. Der Drucksensor spricht dadurch schneller an und ist von etwaigen Schwingungen, die sich in einer vorgelagerten Druckkammer aufbauen könnten, unbeeinflusst. Insbesondere wird das Drucksignal nicht durch das Medium in der Druckkammer beeinflusst.

Dies kann ansonsten durch den Einfluss von Temperatur und Feuchtigkeit sehr leicht geschehen.

Weiter vorteilhaft ist es, wenn als druckempfindliche Membran eine Glasmembran (SiO₂) oder Keramikmembran (z.B. Si₃N₄) dient, die mit ihrer Oberfläche unmittelbar an die Umgebung angrenzt. Die Membran weist auf ihrer der Umgebung zugewandten Oberfläche insbesondere keine zusätzliche Beschichtung auf sondern hat Kontakt zum Blut. Herkömmliche derartige Membranen sind polymerbeschichtet, beispielsweise mit Silikon, und diese Beschichtungen können quellen und/ oder besitzen einen anderen Temperaturausdehnungskoeffizient als die Membran selbst. Dadurch werden Spannungen auf die Membran ausgeübt, die zur Drift des Messergebnisses führen. Indem nun als der Umgebung ausgesetzte druckempfindliche Fläche eine völlig unbeschichtete Membran verwendet wird, können diese Nachteile vermieden und die Messergebnisse dementsprechend weiter verbessert werden.

Je nach den zu messenden Druckereignissen kann die Rotationsblutpumpe mit ein, zwei oder mehr als zwei Drucksensoren ausgestattet sein, die außen an der Blutpumpe, beispielsweise am proximalen und/ oder am distalen Ende der Pumpeinrichtung, und/ oder auch innerhalb der Pumpeinrichtung, beispielsweise in einer Strömungskanüle, vorgesehen sein können.

Vorzugsweise wird ein erster Drucksensor an einem Pumpengehäuse der Pumpeinrichtung fixiert, in dem das Flügelrad oder gegebenenfalls mehrere Flügelräder der Rotationsblutpumpe rotieren. Und zwar besitzt die Pumpeinrichtung zwischen dem Flügelrad und dem Katheter ein oder mehrere Blutströmungsdurchtrittsöffnungen, in deren Nähe die druckempfindliche Fläche angeordnet wird, nämlich vorzugsweise distal von diesen Blutströmungsdurchtrittsöffnungen. Wenn nun der Katheter zu weit durch eine Herzklappe hindurch vorgeschoben wird, so gelangt der Drucksensor in den Bereich der Herzklappe, noch bevor durch weiteres Voranschieben die Blutströmungsdurchtrittsöffnungen von der Herzklappe verschlossen werden. Man stellt sofort fest, wenn die Blutpumpe mit ihrem Pumpenauslass (oder -einlass) in den Bereich der Aortenklappe rutscht. Neben der primären Aufgabe, physiologische Drücke zu messen, die über den Gesundheitszustand des Herzens Aufschluss geben, hat dieser Drucksensor somit die weitere Funktion der korrekten Positionierung der Blutpumpe im Gefäßsystem des Patienten. In diesem Fall kann druckbasiert genau sichergestellt werden, dass sich die Einlassöffnungen in der Herzkammer und die Auslassöffnungen in der Aorta befinden. Hierzu wird bei Verwendung nur eines Drucksensors distal von den Auslassöffnungen die Modulation des Motorstroms der Pumpe bei laufendem Pumpenbetrieb und bei sich kontrahierendem Herzen hinzugezogen. Hierdurch ergibt sich bei Lage der Einlassöffnungen im Herzen und Lage der Auslassöffnungen in der Aorta ein pulsierender Pumpenfluss und folglich auch ein pulsierender Pumpenmotorstrom.

Ergänzend oder alternativ wird ein zweiter Drucksensor am distalen Ende der Pumpeinrichtung angeordnet. Das distale Ende der Pumpeinrichtung besteht in der Regel aus einer Strömungskanüle, mit ein oder mehreren weiteren Blutströmungsdurchtrittsöffnungen für den Eintritt (oder Austritt) des Bluts in die (bzw. aus der) Pumpeinrichtung. Das heißt, durch die Strömungskanüle wird im Betrieb der Blutpumpe Blut von der Pumpeinrichtung angesaugt (oder ausgestoßen). Der Drucksensor kann innerhalb der Strömungskanüle angeordnet sein, um beispielsweise ein Festsaugen der Strömungskanüle an einer Herzkammerwand identifizieren zu können. Wichtig ist aber auch ein Drucksensor außen an der Strömungskanüle in der Nähe ihrer Blutströmungsdurchtrittsöffnungen, um den dortigen physiologischen Druck zu messen. Somit kann gezielt der ventrikuläre Druck mit hoher zeitlicher Auflösung (bis zu 250 Hz) erfasst werden. Hieraus lassen sich die Herzerholung in Form der Herzkontraktilität oder die passive Herzwandspannung indirekt durch Messung der Relaxationsgeschwindigkeit (≅ Beginn der diastolischen Füllphase) in Form der druckbasierten Herzerschlaffung erfassen. Zudem lassen sich die enddiastolischen Fülldrücke erfassen, so dass auch bedarfsgerecht mit der Pumpe Blut entnommen werden kann und hohe Wandspannungen bei hohen enddiastolischen Drücken, die einer Herzerholung entgegenstehen, vermieden werden.

Soweit der Sensorkopf des Drucksensors oder, allgemein ausgedrückt, das distale Ende des Drucksensors außen an der Pumpeinrichtung fixiert wird, also etwa außen am Pumpengehäuse oder außen an der Strömungskanüle, wird der Drucksensor vorzugsweise zumindest über einen Teil der Pumpeinrichtung von proximal nach distal außen an der Pumpeinrichtung entlanggeführt bis zu einer in der äußeren Oberfläche der Pumpeinrichtung vorgesehenen Vertiefung, in der das distale Ende des Drucksensors zumindest teilweise aufgenommen wird. Dadurch werden der empfindliche Sensorkopf und insbesondere dessen druckempfindliche Membran vor einer Kollision mit einem Schleusenventil oder hämostatischen Ventil geschützt, wenn die Blutpumpe ins Gefäßsystem des Patienten eingeführt wird.

Es kann sein, dass die Wandstärke der Pumpeinrichtung nicht ausreicht, um eine Vertiefung mit einer Tiefe herzustellen, in der der Sensorkopf vollständig aufgenommen werden kann, so dass das distale Ende des Drucksensors radial über die Peripherie der Pumpeinrichtung hinausragt. Insbesondere für diese Fälle ist es vorteilhaft, distal vor dem Sensorkopf eine ebenfalls über die Peripherie der Pumpeinrichtung hinausragende Wulst vorzusehen, damit das hämostatische Ventil oder Schleusenventil beim Einführen der Blutpumpe in das Gefäßsystem des Patienten nicht am distalen Ende des Drucksensors hängenbleibt. Besonders bevorzugt ist es in diesem Zusammenhang, diese Wulst zumindest U-förmig oder gegebenenfalls auch vollständig O-förmig um die Vertiefung herumzuführen. Diese Wulst kann beispielsweise durch eine Klebstoffwulst gebildet werden, die gegebenenfalls auch erst nach dem Fixieren des Sensorkopfs in der Vertiefung aufgebracht wird. Die Wulst, insbesondere die U-förmige Wulst, kann alternativ auch aufgeschweißt oder aufgelötet werden oder integraler Bestandteil des Bauteils sein.

Nachfolgend wird die Erfindung anhand der begleitenden Zeichnungen beispielhaft erläutert. Darin zeigen:
Figur 1 eine durch die Aorta verlegte Blutpumpe, die sich durch die Aortenklappe bis in den linken Ventrikel erstreckt, mit integriertem Druck- und Knicksensor,
Figur 2 einen optischen Drucksensor mit Lichtleitfaser,
Figur 3 die Pumpeinrichtung der Blutpumpe aus Figur 1 in größerem Detail,
Figur 4A, 4B den Ausschnitt A aus Figur 3 in Draufsicht und in Seitenansicht und
Figuren 5A, 5B den Ausschnitt B aus Figur 3 in Draufsicht und in Seitenansicht.

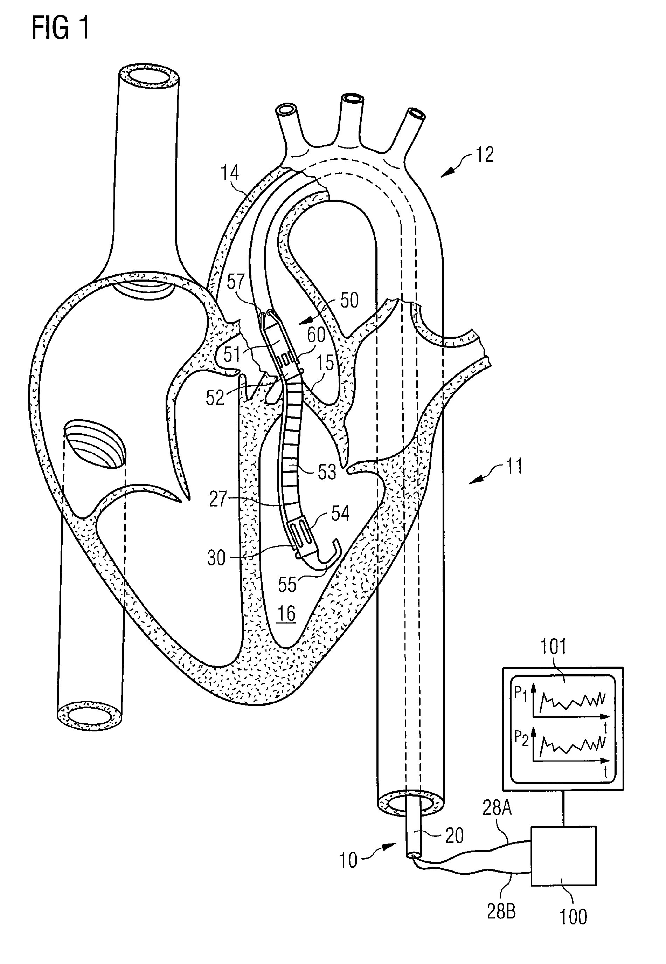

Figur 1 zeigt eine intravasale Blutpumpe mit einem Katheter 10, der retrograd in die Aorta descendens 11 eingeführt ist. Die Aorta descendens ist Bestandteil der Aorta 12, die vom Herzen zunächst aufsteigt und dann abwärts führt und den Aortenbogen 14 aufweist. Am Beginn der Aorta 12 befindet sich die Aortenklappe 15, die den linken Ventrikel 16 mit der Aorta 12 verbindet und durch die hindurch sich die intravasale Blutpumpe erstreckt. Die intravasale Blutpumpe umfasst zusätzlich zu dem Katheter 10 eine am distalen Ende des Katheterschlauchs 20 befestigte rotatorische Pumpeinrichtung 50, die einen Motorteil 51 und einen in axialem Abstand hiervon angeordneten Pumpenteil 52 sowie eine von dem Ansaugende des Pumpenteils 52 in distaler Richtung abstehende Strömungskanüle 53 aufweist, an deren Ende sich ein Saugeinlass 54 befindet. Distal von dem Saugeinlass 54 ist eine weichflexible Spitze 55 vorgesehen, die beispielsweise als "Pigtail" oder in J-Form ausgeführt sein kann. Durch den Katheterschlauch 20 verlaufen verschiedene Leitungen und Einrichtungen, die für den Betrieb der Pumpeinrichtung 50 von Bedeutung sind. Davon sind in Figur 1 lediglich zwei Lichtleitfasern 28A, 28B gezeigt, die mit ihrem proximalen Ende an eine Auswerteeinrichtung 100 angeschlossen sind. Diese Lichtleitfasern 28A, 28B sind jeweils Teil eines optischen Drucksensors, deren Sensorköpfe 30 und 60 sich einerseits außen am Gehäuse des Pumpenteils 52 und andererseits außen am Saugeinlass 54 befinden. Der von den Sensorköpfen 30 und 60 übermittelte Druck wird in der Auswerteeinrichtung 100 in elektrische Signale umgewandelt und z.B. auf einem Display 101 angezeigt.

Durch die Messung sowohl des Aortendrucks mittels des Sensorkopfs 60 als auch des Ventrikeldrucks mittels des Sensorkopfs 30 sind zusätzlich zum eigentlichen Drucksignal z.B. eine Kontraktibilitätsmessung, bei der die Erholung des Herzens gemessen wird, sowie die Ermittlung der Druckdifferenz, die zur Flussberechnung der Pumpeinrichtung 50 hinzugezogen wird, möglich.

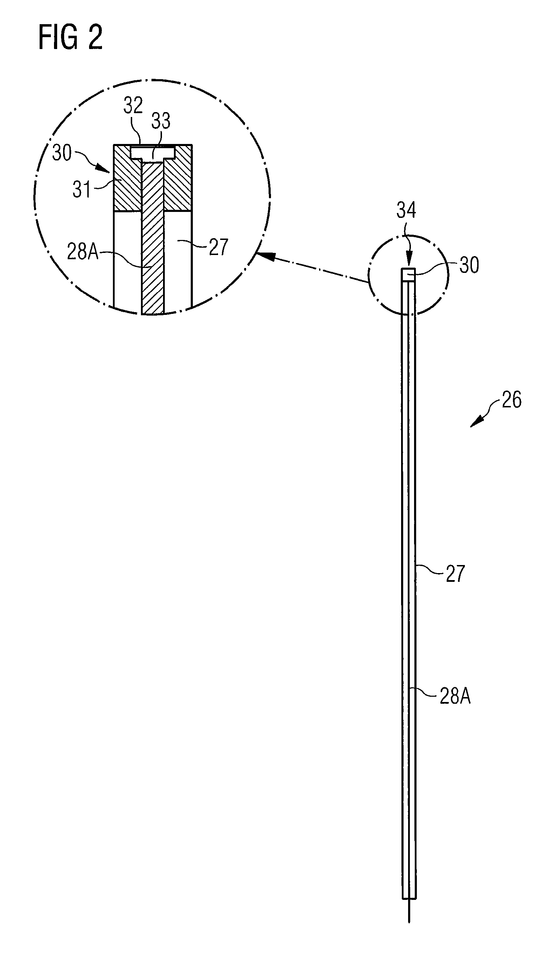

Das Prinzip der elektrooptischen Druckmessung wird nachfolgend anhand der Figur 2 näher erläutert. Figur 2 zeigt einen Druckmesskatheter 26 mit einem Lumen 27, in dem eine Lichtleitfaser 28A (es könnten auch mehrere Lichtleitfasern oder die Lichtleitfaser 28B sein) frei beweglich ist. Das Lumen 27 kann aus einem Polymer, insbesondere Polyurethan, oder vorzugsweise aus Nitinol oder einer anderen Formgedächtnislegierung bestehen, an einer Austrittsstelle 57 aus dem Katheterschlauch 20 austreten (vgl. Figur 1) und an der flexiblen Strömungskanüle 53 z.B. außen entlang verlegt sein. Innerhalb des Katheterschlauchs 20 kann auf das separate Lumen 27 verzichtet werden. Am distalen Ende 34 der Lichtleitfaser 28A weist der Druckmesskatheter einen Sensorkopf 30 mit einem Kopfgehäuse 31 auf, das eine dünne Keramik- oder Glasmembran 32 enthält, die eine Kavität 33 abschließt. Die Membran 32 ist druckempfindlich und verformt sich in Abhängigkeit von der Größe eines auf den Sensorkopf 30 einwirkenden Drucks. Durch die Reflexion an der Membran wird das aus der Lichtleitfaser 28A austretende Licht modulierend reflektiert und wieder in die Lichtleitfaser eingekoppelt. Dazu ist es nicht notwendig, dass die Kavität 33 mit der Lichtleitfaser 34 abgeschlossen wird. Dies kann ebenfalls durch das Kopfgehäuse 31 erfolgen. Es ist lediglich auf ein verlustarmes Ein- und Auskoppeln des Lichts zu achten. Am proximalen Ende der Lichtleitfaser 28A, d. h. in der Auswerteeinrichtung 100, befindet sich eine Digitalkamera, z.B. eine CCD-Kamera oder ein CMOS, die das eintreffende Licht in Form eines Interferenzmusters auswertet. In Abhängigkeit davon wird ein druckabhängiges elektrisches Signal erzeugt. Die Auswertung des von der Kamera gelieferten optischen Bildes bzw. optischen Musters und die Berechnung des Drucks erfolgen durch einen an die Kamera angeschlossenen Rechner, der auch die Stromzufuhr zu der motorisch betriebenen Pumpeinrichtung 50 in Abhängigkeit von der erfolgten Auswertung des Drucksignals steuert.

Anstelle des in Bezug auf Figur 2 beschriebenen optischen Drucksensors, der nach dem Fabri-Perot-Prinzip arbeitet, können auch andere Drucksensoren, insbesondere optische Drucksensoren mit ein oder mehreren Lichtleitfasern, verwendet werden, vorausgesetzt dass diese anderen Drucksensoren eine orthogonal zur Längsachse der Pumpeinrichtung 50 ausgerichtete und der Umgebung ausgesetzte druckempfindliche Fläche besitzen, beispielsweise eine Membran oder ein andersartiges Diaphragma.

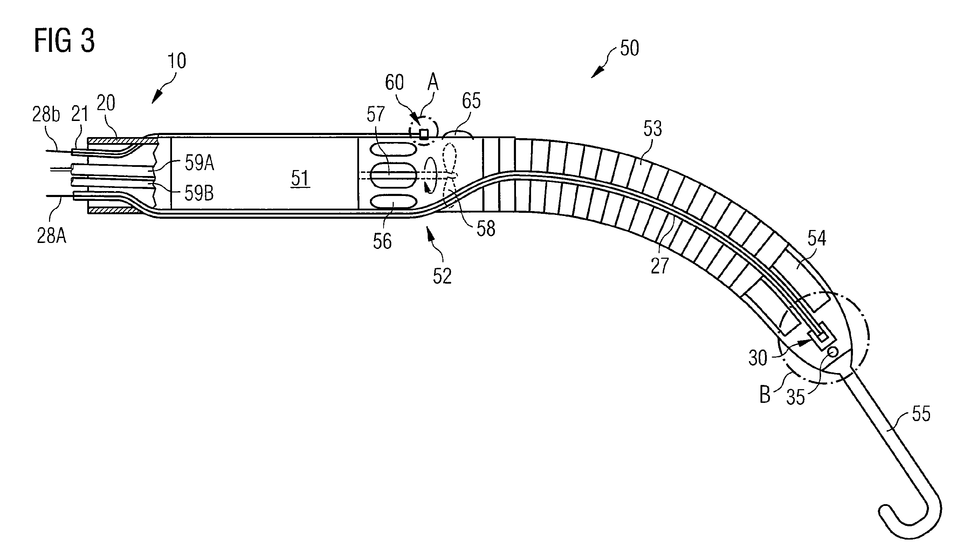

Die Pumpeinrichtung 50 aus Figur 1 ist in Figur 3 in weiterem Detail dargestellt. Zu sehen ist eine vom Motorteil 51 in den Pumpenteil 52 hineinragende Antriebswelle 57, welche ein Flügelrad 58 antreibt, mittels dessen im Betrieb der Blutpumpe Blut durch die Blutdurchtrittsöffnungen 54 am distalen Ende der flexiblen Strömungskanüle 53 hindurch angesaugt und proximal von dem Flügelrad 58 durch die Blutströmungsdurchtrittsöffnungen 56 ausgestoßen wird. Die Pumpeinrichtung 50 kann auch in umgekehrter Richtung fördern, wenn sie entsprechend angepasst wird. Durch den Katheterschlauch 20 des Katheters 10 hindurch zur Pumpeinrichtung 50 führen einerseits die bereits erwähnten Lichtleitfasern 28A, 28B sowie eine Stromversorgungsleitung 59A für den Motorteil 51 und eine Spülflüssigkeitsleitung 59B.

Der Sensorkopf 60 des ersten Drucksensors ist außen am Pumpengehäuse des Pumpenteils 52 fixiert. Die zugehörige Lichtleitfaser 28B ist innerhalb des Katheterschlauchs 20 über eine kurze Distanz von beispielsweise 5 cm in einem dünnen Kunststoffschlauch 21 geführt, um bei starken Krümmungen des Katheters 10 in diesem Bereich des Katheterschlauchs 20 sicherzustellen, dass die Lichtleitfaser 28B nicht bricht. Außerhalb der Pumpeinrichtung 50 ist die Lichtleitfaser 28B frei verlegt und lediglich mittels Klebstoff an der Außenwand der Pumpeinrichtung 50 verklebt. Dadurch werden die äußeren Querschnittsabmessungen der Pumpeinrichtung 50 minimal gehalten. Das Verkleben der Lichtleitfaser 28B ist möglich, weil die Pumpeinrichtung 50 in diesem Bereich starr ist und die Lichtleitfaser 28B sich daher nicht relativ zur Pumpeinrichtung 50 bewegen können muss.

Demgegenüber ist die zum Sensorkopf 30 des zweiten Drucksensors führende Lichtleitfaser 28A entlang der gesamten Peripherie der Pumpeinrichtung 50 in einem Schlauch oder Röhrchen 27, vorzugsweise einem Nitinol-Röhrchen, frei verlegt, so dass sie sich innerhalb dieses Schlauchs bzw. Röhrchens bei Biegungsänderungen der Strömungskanüle 53 relativ zur Pumpeinrichtung 50 verlagern kann.

Der Schlauch und/ oder das Röhrchen 27, in denen die Lichtleitfasern 28A, 28B verlegt sind, können sich bis kurz in den Katheterschlauch 20 hinein erstrecken, können sich aber auch durch den Katheterschlauch 20 vollständig hindurch erstrecken und in einem entsprechenden Stecker am Ende der Leitung zum Einstecken des betreffenden Drucksensors in einen Anschluss der Auswerteeinrichtung 100 enden. Die Lichtleitfaser 28B wie auch die Lichtleitfaser 28A sind vorzugsweise Glasfasern, die üblicherweise zu ihrer Isolation polymerbeschichtet sind, beispielsweise mit Polyimid (Kapton).

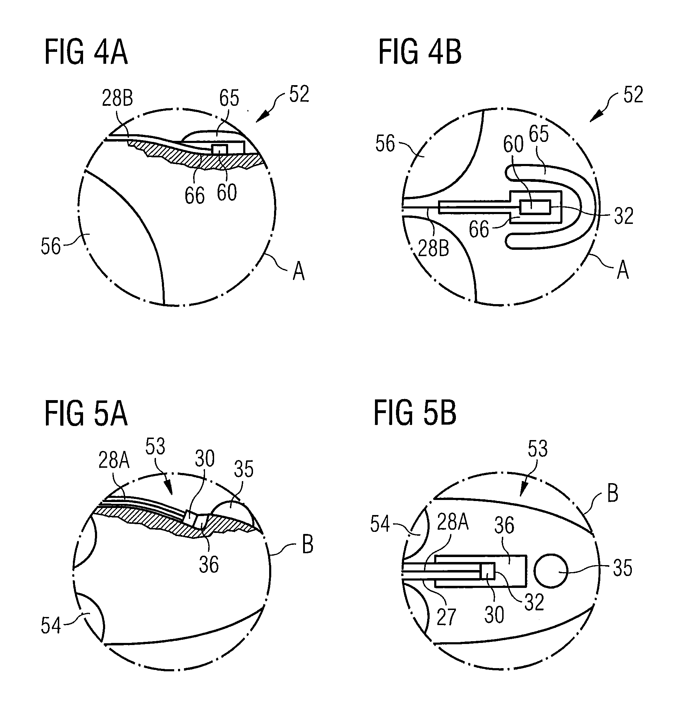

Distal vor den Sensorköpfen 30 und 60 ist jeweils eine Wulst 35 bzw. 65 vorgesehen, welche die Sensorköpfe 30 und 60 beim Einführen der Blutpumpe durch ein hämostatisches Ventil oder Schleusenventil hindurch vor Beschädigung schützen. Darüber hinaus sind die Sensorköpfe 30 und 60 jeweils in eine Vertiefung 36 bzw. 66 der Pumpeinrichtung 50 eingelassen. Letzteres ist in Figur 3 nicht dargestellt und wird nachfolgend in Bezug auf die Figuren 4A, 4B und 5A, 5B erläutert.

Figur 4A zeigt den Ausschnitt A aus Figur 3 in größerem Detail und teilweise im Querschnitt. Figur 4B zeigt im Grunde den gleichen Ausschnitt A jedoch in Aufsicht von oben. Demnach ist der Sensorkopf 60 in einer auf der äußeren Oberfläche des Pumpenteils 52 vorgesehenen Vertiefung 66 versenkt aufgenommen, wobei die Vertiefung 66 von einer Hufeisen- oder U-förmigen Wulst 65 umgeben ist. Die Wulst könnte auch zu einer O-Form geschlossen sein. Sie ist aufgeklebt oder aufgeschweißt, kann aber auch einen integralen Bestandteil des Pumpenteils 52 bilden. Die Lichtleitfaser 28B ist auf der Oberfläche verklebt und verläuft entlang eines Stegs zwischen zwei Blutströmungsdurchtrittsöffnungen 56.

In ähnlicher Weise ist auch der Sensorkopf 30 des zweiten Drucksensors in einer Vertiefung 36 auf der äußeren Oberfläche am distalen Ende der Strömungskanüle 53 versenkt aufgenommen. Auch hier verläuft das Nitinol-Röhrchen 27 mit der darin verlegten Lichtleitfaser 28A über einen Steg zwischen zwei Blutströmungsdurchtrittsöffnungen 54 hindurch. Eine punktförmige Wulst 35 distal unmittelbar vor der Vertiefung 36 schützt den Sensorkopf 30 beim Einführen der Blutpumpe vor Kollisionsbeschädigung. Auch die Wulst 35 kann alternativ U-förmig oder O-förmig ausgebildet und insbesondere aufgeklebt, aufgeschweißt oder ein integraler Bestandteil der Strömungskanüle 53 sein.

Zu sehen an diesen beiden Ansichten gemäß Figur 4A, 4B sowie 5A, 5B ist die orthogonale Ausrichtung der jeweiligen druckempfindlichen Fläche bzw. Keramik- oder Glasmembran 32 relativ zur Längsachse der Pumpeinrichtung 50.

Der Sensorkopf 30 kann alternativ zusammen mit dem Schlauch oder Röhrchen 27 bis an eine beliebige Stelle der weichflexiblen Spitze 55 reichen und dort z.B. durch die Bewandung der weichflexiblen Spitze 55 mechanisch geschützt sein. Biegungsinduzierte Druckartefakte sind gering, da die Sensormembran orthogonal zur Bewandung angeordnet ist. Lediglich die Klebeverbindung zwischen dem Lichtwellenleiter 34 und dem Sensorkopf 30 muss gegen Verbiegung geschützt werden. Dies kann durch das Röhrchen 27 oder eine zusätzliche Versteifung im Bereich der Klebung erfolgen.

## Patentansprüche

1. Intravasale Rotationsblutpumpe umfassend einen Katheter (10), eine distal am Katheter (10) fixierte Pumpeinrichtung (50) mit einer allgemeinen Längsachse und mindestens einen fest mit der Pumpeinrichtung (50) verbundenen Drucksensor (27,28A, 30; 28B, 60) mit einer druckempfindlichen Fläche (32), wobei die druckempfindliche Fläche (32) der Umgebung ausgesetzt ist und **dadurch gekennzeichnet, dass** die druckempfindliche Fläche (32) orthogonal zu der allgemeinen Längsachse ausgerichtet ist.

2. Blutpumpe nach Anspruch 1, wobei der mindestens eine Drucksensor an einem Pumpengehäuse der Pumpeinrichtung (50) fixiert ist, in welchem mindestens ein Flügelrad (58) rotiert.

3. Blutpumpe nach Anspruch 2, umfassend mindestens eine erste Blutströmungsdurchtrittsöffnung (56) zwischen dem Flügelrad (58) und dem Katheter (10), wobei der Drucksensor so an dem Pumpengehäuse fixiert ist, dass die druckempfindliche Fläche (32) distal von und nahe bei der ersten Blutströmungsdurchtrittsöffnung (56) angeordnet ist.

4. Blutpumpe nach einem der Ansprüche 1 bis 3, wobei die Pumpeinrichtung (50) an ihrem distalen Ende eine Strömungskanüle (53) mit mindestens einer zweiten Blutströmungsdurchtrittsöffnung (54) aufweist, durch die hindurch im Betrieb der Blutpumpe Blut von der Pumpeinrichtung (50) entweder angesaugt oder ausgestoßen wird, wobei der mindestens eine Drucksensor nahe bei dieser zweiten Blutströmungsdurchtrittsöffnung (54) angeordnet ist.

5. Blutpumpe nach Anspruch 4, wobei die Strömungskanüle (53) distal von der zweiten Blutströmungsdurchtrittsöffnung (54) eine weichflexible Spitze (55) aufweist und wobei ein distales Ende (30) des Drucksensors zumindest teilweise in der weichflexiblen Spitze (55) angeordnet ist.

6. Blutpumpe nach einem der Ansprüche 1 bis 5, wobei der Drucksensor außen an der Pumpeinrichtung (50) von proximal nach distal entlang geführt ist, und wobei die Pumpeinrichtung (50) eine äußere Oberfläche mit einer Vertiefung (36; 66) aufweist, in der ein distales Ende (30; 60) des Drucksensors zumindest teilweise angeordnet ist.

7. Blutpumpe nach einem der Ansprüche 1 bis 6, wobei ein distales Ende (30; 60) des Drucksensors über die Peripherie der Pumpeinrichtung (50) radial hinausragt, und wobei an der Pumpeinrichtung (50) distal vor diesem Ende (30; 60) des Drucksensors eine ebenfalls über die Peripherie der Pumpeinrichtung (50) hinausragende Wulst (35; 65) vorgesehen ist.

8. Blutpumpe nach Anspruch 7, wobei die Wulst (35; 65) U-förmig oder O-förmig ist.

9. Blutpumpe nach Anspruch 7 oder 8, wobei die Wulst (35; 65) eine Klebstoffwulst ist.

10. Blutpumpe nach Anspruch 7 oder 8, wobei die Wulst (35; 65) auf eine Oberfläche der Pumpeinrichtung (50) aufgeschweißt oder aufgelötet ist.

11. Blutpumpe nach Anspruch 7 oder 8, wobei die Wulst (35; 65) einen integralen Bestandteil der Pumpeinrichtung (50) bildet.

12. Blutpumpe nach einem der Ansprüche 1 bis 11, wobei der Drucksensor ein optischer Drucksensor mit einer Lichtleitfaser (28A; 28B) ist, und wobei die druckempfindliche Fläche (32) eine Membran ist und die Lichtleitfaser (28A, 28B) in einem Abstand zur Membran endet.

13. Blutpumpe nach einem der Ansprüche 1 bis 12, wobei die druckempfindliche Fläche (32) eine Keramik- oder Glasmembran ist, die mit ihrer Keramik- bzw. Glasoberfläche unmittelbar der Umgebung ausgesetzt ist.

## Claims

1. An intravascular rotation blood pump comprising a catheter (10), a pumping device (50) with a general longitudinal axis distally fixed to the catheter (10) and at least one pressure sensor (27, 28A, 30; 28B, 60) firmly connected to the pumping device (50) and having a pressure-sensitive area (32),
wherein the pressure-sensitive area (32) is subjected to the environment and **characterized in that** the pressure-sensitive area (32) is oriented orthogonally to the general longitudinal axis.

2. The blood pump according to claim 1, wherein the at least one pressure sensor is fixed to a pump housing of the pumping device (50), with at least one impeller (58) rotating in the former.

3. The blood pump according to claim 2, comprising at least one first blood-stream passage opening (56) between the impeller (58) and the catheter (10), wherein the pressure sensor is so fixed to the pump housing that the pressure-sensitive area (32) is arranged distally of and near the first blood-stream passage opening (56).

4. The blood pump according to any of the claims 1 to 3, wherein the pumping device (50) has at its distal end a stream cannula (53) with at least one second blood-stream passage opening (54), passing through which blood is either drawn in or discharged by the pumping device (50) during the operation of the blood pump, wherein the at least one pressure sensor is arranged near this second blood-stream passage opening (54).

5. The blood pump according to claim 4, wherein the stream cannula (53) has a soft flexible tip (55) distally of the second blood-stream passage opening (54), and wherein a distal end (30) of the pressure sensor is arranged at least partially in the soft flexible tip (55).

6. The blood pump according to any of the claims 1 to 5, wherein the pressure sensor is guided along the outside of the pumping device (50) from proximal to distal, and wherein the pumping device (50) has an outer surface with a depression (36; 66) in which a distal end (30; 60) of the pressure sensor is arranged at least partially.

7. The blood pump according to any of the claims 1 to 6, wherein a distal end (30; 60) of the pressure sensor radially projects beyond the periphery of the pumping device (50), and wherein on the pumping device (50), distally in front of this end (30; 60) of the pressure sensor, a bead (35; 65) is provided which likewise projects beyond the periphery of the pumping device (50).

8. The blood pump according to claim 7, wherein the bead (35; 65) is U-shaped or O-shaped.

9. The blood pump according to claim 7 or 8, wherein the bead (35; 65) is an adhesive bead.

10. The blood pump according to claim 7 or 8, wherein the bead (35; 65) is welded or soldered onto a surface of the pumping device (50).

11. The blood pump according to claim 7 or 8, wherein the bead (35; 65) forms an integral part of the pumping device (50).

12. The blood pump according to any of the claims 1 to 11, wherein the pressure sensor is an optical pressure sensor having an optical fiber (28A; 28B), and wherein the pressure-sensitive area (32) is a membrane and the optical fiber (28A, 28B) ends at a distance to the membrane.

13. The blood pump according to any of the claims 1 to 12, wherein the pressure-sensitive area (32) is a ceramic or glass membrane which is subjected directly to the environment with its ceramic or glass surface.

## Revendications

1. Pompe à sang rotative intravasculaire comprenant un cathéter (10), un dispositif de pompage (50) fixé sur le cathéter (10) de manière distale présentant un axe généralement longitudinal et au moins un capteur de pression (27, 28A, 30; 28B, 60) doté d'une surface sensible à la pression (32) et relié de manière permanente au dispositif de pompage (50), dans laquelle la surface sensible à la pression (32) est exposée à l'environnement, et **caractérisée en ce que** la surface sensible à la pression (32) est orientée de manière orthogonale par rapport à l'axe généralement longitudinal.

2. Pompe à sang selon la revendication 1, dans laquelle le au moins un capteur de pression est fixé sur un corps de pompe du dispositif de pompage (50), dans lequel tourne au moins une roue à ailettes (58).

3. Pompe à sang selon la revendication 2, comprenant au moins une première ouverture de passage pour débit sanguin (56) entre la roue à ailettes (58) et le cathéter (10), dans laquelle le capteur de pression est fixé sur le corps de pompe de telle sorte que la surface sensible à la pression (32) soit agencée de manière distale par rapport à la première ouverture de passage pour débit sanguin (56), et à proximité de celle-ci.

4. Pompe à sang selon l'une quelconque des revendications 1 à 3, dans laquelle le dispositif de pompage (50) présente sur son extrémité distale une canule d'écoulement (53) dotée d'au moins une seconde ouverture pour débit sanguin (54), à travers laquelle lors du fonctionnement de la pompe à sang, du sang est soit aspiré soit refoulé par le dispositif de pompage (50), le au moins un capteur de pression étant agencé à proximité de ladite seconde ouverture de passage pour débit sanguin (54).

5. Pompe à sang selon la revendication 4, dans laquelle la canule d'écoulement (53) présente une pointe souple (55) de manière distale par rapport à l'ouverture de passage pour débit sanguin (54), et dans laquelle une extrémité distale (30) du capteur de pression est agencée au moins en partie dans la pointe souple (55).

6. Pompe à sang selon l'une quelconque des revendications 1 à 5, dans laquelle le capteur de pression est guidé à l'extérieur sur le dispositif de pompage (50) de manière longitudinale du côté proximal au côté distal, et dans laquelle le dispositif de pompage (50) présente une surface extérieure dotée d'un évidement (36; 66), dans lequel est agencée au moins en partie une extrémité distale (30; 60) du capteur de pression.

7. Pompe à sang selon l'une quelconque des revendications 1 à 6, dans laquelle une extrémité distale (30; 60) du capteur de pression fait saillie de manière radiale au-dessus de la périphérie du dispositif de pompage (50), et dans laquelle est prévu un bourrelet (35; 65) faisant également saillie au-dessus de la périphérie du dispositif de pompage (50), sur le dispositif de pompage (50) et de manière distale devant ladite extrémité (30; 60) du capteur de pression.

8. Pompe à sang selon la revendication 7, dans laquelle le bourrelet (35; 65) est un bourrelet en forme de U ou en forme de O.

9. Pompe à sang selon la revendication 7 ou 8, dans laquelle le bourrelet (35; 65) est un bourrelet de colle.

10. Pompe à sang selon la revendication 7 ou 8, dans laquelle le bourrelet (35; 65) est soudé ou brasé sur une surface du dispositif de pompage (50).

11. Pompe à sang selon la revendication 7 ou 8, dans laquelle le bourrelet (35; 65) fait partie intégrante du dispositif de pompage (50).

12. Pompe à sang selon l'une quelconque des revendications 1 à 11, dans laquelle le capteur de pression est un capteur de pression optique doté d'une fibre optique (28A; 28B), et dans laquelle la surface sensible à la pression (32) est une membrane, et la fibre optique (28A, 28B) se termine à une distance de la membrane.

13. Pompe à sang selon l'une quelconque des revendications 1 à 12, dans laquelle la surface sensible à la pression (32) est une membrane en céramique ou en verre qui est exposée directement à l'environnement sur sa surface en céramique ou en verre.
